# EUROPEAN PATENT SPECIFICATION

(11) **EP 1 842 064 B1**
(45) Date of publication and mention of the grant of the patent: **25.12.2013**
(21) Application number: 06719248.4
(22) Date of filing: 24.01.2006
(51) Int. Cl.: G01N 33/53, G01N 33/537, G01N 33/543, G01N 33/566

(54) **ISCHEMIC BIOMARKERS AND THEIR USE TO PREDICT ADVERSE NEUROLOGICAL EVENTS FROM SUGERY**
ISCHÄMISCHE BIOMARKER UND DEREN VERWENDUNG ZUR VORHERSAGE UNERWÜNSCHTER NEUROLOGISCHER EREIGNISSE NACH EINEM CHIRURGISCHEN EINGRIFF
BIOMARQUEURS ISCHEMIQUES ET UTILISATION ASSOCIEE POUR PREVOIR DES EVENEMENTS NEUROLOGIQUES INDESIRABLES DUS A UNE INTERVENTION CHIRURGICALE

(30) Priority: 25.01.2005 US 646762 P
(43) Date of publication of application: 10.10.2007
(73) Proprietor: Cis Biotech, Inc., Atlanta, GA 30329 (US)
(72) Inventor: DAMBINOVA, Svetlana, A., Atlanta, Georgia 30329 (US)
(74) Representative: Leissler-Gerstl, Gabriele
(86) International application number: PCT/US2006/002306
(87) International publication number: WO 2006/081188

(56) References cited:
- WO-A-03/011271
- WO-A2-02/12892
- US-A1- 2003 096 331
- DAMBINOVA ET AL.: 'Multiple Panel of Biomarkers for TIA/Stroke Evaluation' STROKE vol. 33, no. 5, 2002, pages 1181 - 1182, XP003003586
- DAMBINOVA ET AL.: 'Blood Tests Detecting Autoantibodies to N-Methyl-D-aspartate Neuroreceptors for Evaluation of Patients with Transiest Ischemic Attack and Stroke' CLINICAL CHEMISTRY vol. 49, no. 10, 2003, pages 1752 - 1762, XP003003587
- DAMBINOVA ET AL.: 'Autoantibodies to Subtypes of Glutamate Receptors as a Hallmrk of Brain Damage: Diagnostic Significance for Paroxysmal Activity and Ischemia' JOURNAL OF HIGHER NERVOUS ACTIVITY vol. 47, no. 2, 1997, pages 151 - 156, XP008082478

## Description

### RELATION TO PRIOR APPLICATIONS

This application claims priority under 35 U.S.C. § 119(e) to United States Provisional Application No. 60/646,762, filed January 25, 2005.

### FIELD OF THE INVENTION

The present invention relates to methods for predicting the risk of adverse neurological events from surgery. In particular, the present invention relates to methods of testing for NR2 peptides and antibodies in the blood of patients scheduled for surgery, and to methods of using such test results to predict the likelihood of a stroke, transient ischemic attack (TIA) or other ischemic induced episode in a patient from surgery. This testing is particularly useful in patients with preexisting cardiovascular disorders, cerebrovascular disorders, hypertension, diabetes, or carotid bruit, who are more likely to suffer from adverse neurological events from surgery, but whose risk from the surgery is not fully known. The diagnostic and prognostic capabilities of this testing promise to reduce morbidity and mortality in the operating room, and to improve the management of cardio- and cerebrovascular patients assigned to surgery.

### BACKGROUND OF THE INVENTION

Damage to the nervous system from surgery is a combination of direct toxic effects on neurons and secondary damage from systemic hypoxia and ischemia. Strokes are either occlusive (due to blockage of a blood vessel) or hemorrhagic (due to bleeding from a vessel), and both can result in insufficient blood supply to the brain resulting in a condition known as ischemia.

Focal brain injury is different than the global anoxic brain damage that often occurs after cardiac arrest or hypoperfusion (Graham SH, Chen J. J Cereb Blood Flow 2001; 21:99-109). When focal brain injury occurs, an ischemic core develops that is typically surrounded by a penumbra zone of surviving cells. These surviving cells prevent (or at least delay) expansion of the infarct and the amount of brain damage (Deshpande J, et al. Exp Brain Res 1992; 88: 91-105; Matsui T, et al. J Cereb Blood Flow Metab 2002; 22:711-722). When cerebral ischemia occurs globally, the most vulnerable areas of the brain are situated in the parietal cortex and basal ganglia where apoptotic events are observed (Kjos BO, Brant-Zawadzki M, Young RG. Am J Roentgenol 1983; 141:1227-1232).

During the last decade several biomarkers to predict and diagnose brain injury have been proposed. Great interest in serum S100B as a biomarker for neurological and neurocognitive outcome evaluation in cardiac surgery was initiated by reports that S100B correlated with brain damage after stroke, traumatic brain injury, and cardiac arrest (Abraha HD, et al. Ann Clin Biochem 1997; 34: 366-370; Buttner T, et al. Stroke 1997; 28: 1961-1965; Lynch JR, et al. Stroke 2004;35:57-63). S100B is a calcium-regulating protein found primarily in glia and Schwann cells. Another possible biomarker for cerebrovascular events from cardiac surgery is C-reactive protein (CRP), an acute-phase reactant and indicator of underlying systemic inflammation. CRP is a novel plasma marker for atherothrombotic disease and predictor of cardiovascular disease (Rost NS, et al. Stroke 2001; 32:2575-79).

Recently, N-methyl-D-aspartate (NMDA) receptor peptides and their antibodies have been proposed as biomarkers of neurotoxicity underlying cerebral ischemia and stroke (Dambinova SA, et al. Stroke 2002; 33:1181-1182; Dambinova SA, et al. Clin Chem 2003; 49:1752-1762). The NMDA receptor is unique to the brain. With neuronal death or ischemia, peptide fragments of the NMDA receptor break off and appear in the bloodstream and generate an antibody response. The peptide fragment and antibody can both be detected in blood samples (Dambinova SA, et al. Stroke 2002). Adult patients who suffer from acute ischemic stroke have elevated blood levels of NMDA peptide and/or antibodies that correlate with the amount of brain damage on brain scans (MRI) and the patient's neurocognitive status (Dambinova SA, et al. Clin Chem 2003; 49:1752-1762).

The routine application of specific biomarkers with potential diagnostic and prognostic capabilities could greatly improve the management and therapeutic outcome of cardio- and cerebrovascular patients undergoing surgery, especially heart surgery involving cardiopulmonary bypass (CPB). Cerebral complications represent the leading cause of morbidity and disability after cardiac surgery with cardiopulmonary bypass. The incidence of stroke ranges between 1 and 5% during the perioperative period (Gardner TJ, et al. Ann Thorac Surg 1985; 40:574-581; Murkin JM, et al. J Thorac Cardiovasc Surg 1995; 110:349-362; Roach GW, et al. N Engl J Med 1996; 335:1857-1863; Libman RB, et al. Arch Neurol 1997; 54:83-87; Carrascal Y, et al. Eur Neurol 1999; 41:128-134), and its occurrence is associated with an increased mortality (Roach GW, et al. N Engl J Med 1996; 335:1857-1863; Carrascal Y, et al. Eur Neurol 1999; 41:128-134). Although stroke is an obvious complication, neurocognitive dysfunction and confusion are more subtle complications. The impact of neurocognitive dysfunction on postoperative care, and the cost associated with prolonged hospitalization from such dysfunction, are enormous.

Global cerebral ischemia is associated with cardiovascular disorders and greatly contributes to worsening of neurocognitive abilities and neurological complications. Numerous studies have reported minor neuropsychological impairment using a battery of neurocognitive tests, frequently after heart surgery (Murkin JM, et al. J Thorac Cardiovasc Surg 1995; 110:349-362; Shaw PJ, et al. Q J Med 1987; 239:259-268; McKhann GM, et al. Ann Throrac Surg 1997; 63: 510-515; McKhann et al. Lancet 1997; 349:1282-1284). However neurocognitive tests alone are not able to predict adverse neurological events from heart surgery.

Defining cerebral dysfunction determinants associated with cardiac surgery, based on preoperative risk factors and mechanisms linked with operative procedures, is important to reduce the risk from such procedures and to improve patient outcomes. Cerebral damage due to cardiac surgery is currently assessed retrospectively by comparing preoperative and postoperative clinical and neuropsychological evaluations, or using imaging techniques (CT or MRI) to detect morphological changes. Unfortunately, the neuropsychological assessment requires time, expert staff and patient cooperation. In addition, neuroimaging (particularly MRI) is very costly and may be stressful and risky for the patients in the postoperative period. A biochemical marker able to detect cerebral injury and predict further cerebral injury would have considerable practical value.

### OBJECTS OF THE INVENTION

One object of the present invention to accurately predict the risk of adverse neurological events from a planned surgery, including transient ischemic attack (TIA), stroke, and neurocognitive dysfunction, using biomarkers that can be detected in human blood or other biological fluids before the surgery occurs.

Another object of the present invention is to evaluate the risk of ischemia-induced adverse neurological events from a planned surgery in patients at particular risk for such adverse events, including patients with preexisting cardiovascular or cerebrovascular damage or chronic ischemic stress, or diabetes.

Still another object of the invention is to enable a more effective selection of interventional strategies for reducing the risk of adverse neurological events from surgery, including monitoring regimens and neuroprotective therapy against damage caused by cerebral ischemia, before, during and after a surgery.

### SUMMARY OF THE INVENTION

Methods and kits are provided for assessing the risk of a patient suffering an adverse neurological event from surgery based on the presence and amount of NMDA receptor peptides and antibodies in the bloodstream of the patient. Clinically predetermined cut-offs for NMDA receptor peptides and antibodies have allowed us to demonstrate the high-performance characteristics and clinical utility of these peptide and antibody tests for assessing the risk of adverse neurological events, including TIA and stroke, in adult patients before surgery. Analyses of pre- and post-operative NMDA peptide and antibody distributions have shown that these markers have high predictive value for neurological complications alone and when combined with MMSE (Mini-Mental Status Exam) component scores. Diagnostic and therapeutic methods for managing and reducing such risk based on the results of this testing have also been developed.

Blood levels of the NR2A and NR2B subunits of the NMDA receptor, especially peptide fragments from the N-terminal domain of the NR2A and NR2B subunits, are particularly preferred in the methods of the present invention, and remarkably accurate for predicting the likelihood of adverse neurological events from surgery. In one prospective, blinded, multi-center clinical study that measured the incidence of stroke or TIA in patients who underwent cardiopulmonary bypass surgery, the methods were able to predict 96.2% of the patients who would suffer a stroke or TIA when greater than 2.0 ng/ml of antibody was present in patients' blood serum, and 95.6% of the patients who would not suffer a stroke or TIA when less than 2.0 ng/ml of antibodies was present in patients' blood serum. In contrast, S100B, a calcium regulating protein which has been widely investigated as a potential biomarker for neurological outcome in cardiac surgery, had no meaningful ability to predict patients who would or would not suffer a TIA or stroke. CRP (C-reactive protein), which is also reported to be a marker for atherothrombotic disease and a predictor of cerebro-vascular disease, also had no meaningful ability to predict patients who would or would not suffer a TIA, or stroke.

Therefore, in one embodiment the invention provides a method for aiding in the assessment of the risk of stroke in an apparently healthy human subject prior to surgery comprising: (a) obtaining a test sample from the human subject; (b) analyzing the test sample for the presence or amount of an NR2 antigen of an NR2 antibody, or a combination thereof; and comparing the result of step (b) with a corresponding reference amount of an NR2 antigen or NR2 antibody, or a combination thereof, wherein the corresponding reference amount is derived from a population of apparently healthy human subjects.

When an unacceptable risk of TIA or stroke is observed, the invention further provides monitoring regimens and neuroprotective therapies for managing or reducing such risk. For example, when a dangerous level of NMDA receptor peptides or antibodies is observed, the patient could take various drugs known to benefit the cardiovascular system and reduce the risk of stroke, including various antiplatelet agents, anticoagulants, lipid lowering agents, blood pressure medications (if high blood pressure is observed), and surgical intervention. Alternatively or in addition, a strict monitoring regimen could be instituted in which NR2 levels are monitored one or more additional times, including immediately before the surgery, during the surgery, or immediately after the surgery.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### DETAILED DESCRIPTION OF THE INVENTION

The present invention may be understood more readily by reference to the following detailed description of preferred embodiments of the invention and the Examples included therein.

### Definitions and Use of Terms

As used in this specification and in the claims which follow, the singular forms "a," "an" and "the" include plural referents unless the context clearly dictates otherwise. Thus, for example, reference to "a fragment" includes mixtures of fragments, reference to "a cDNA oligonucleotide" includes more than one oligonucleotide, and the like.

An NMDA receptor is one of a family of ligand-gated ion channels that bind preferentially to N-methyl-D-aspartate and that mediate the vast majority of excitatory neurotransmissionin the brain (Dingledine R. et al., Pharmacol Rev. 1999 Mar;51(1):7-61.). The receptors include several subunits reported in the literature as NR1, NR2A, NR2B, NR2C, NR2D and NR3A, that perform distinct pharmacological functions. GenEMBL Accession Nos. have been reported for NR1 (X58633), NR2A (U09002) and NR2B (U28861), and are described in WO 02/12892 to Dambinova. A "cerebral" NMDA receptor refers to a receptor that is present in the brain, as opposed to an NMDA receptor that is only present in an organ of the human body outside of the brain.

An NMDA receptor peptide refers to a full length NMDA receptor protein, a peptide fragment of the naturally occurring full length NMDA receptor, or an analogue, derivative, fragment or recombined (a/k/a recombinant) plurality of fragments thereof. An NR2 peptide includes the full length NR2A, NR2B, NR2C and NR2D subunits, in addition to fragments, analogs, derivatives, and recombined fragments thereof. An NR2A, NR2B, NR2C, or NR2D peptide means the full length naturally occurring NR2A, NR2B, NR2C or NR2D peptide subunits, or a fragment, analog, derivative or recombined fragments thereof. The N-terminal domain of the NR2A and NR2B peptides refers to the amino acid N-terminal domain fragment of the full length NR2A and NR2B subunits, or a fragment, analog, derivative or recombined fragments thereof, as described in WO 02/12892 to Dambinova.

A circulating NMDA receptor peptide refers to an NMDA receptor peptide that has crossed the blood brain barrier into the systemic blood circulation, and fragments thereof generated in the blood stream. A particularly preferred circulating NMDA receptor peptide is one which comprises one or more fragment sequences that are common to both NR2A and NR2B subunits. The peptide may include one such sequence, or 2 or more such sequences that are present at discreet locations on the native NR2 peptide backbone, and recombined into one sequential fragment either in vivo or vitro.

An "analogue" of a peptide means a peptide that contains one or more amino acid substitutions, deletions, additions, or rearrangements. For example, it is well known in the art of protein biochemistry that an amino acid belonging to a grouping of amino acids having a particular size or characteristic (such as charge, hydrophobicity, and hydrophilicity) can often be substituted for another amino acid without altering the activity of the protein, particularly in regions of the protein that are not directly associated with biological activity. Thus, an analogue of an NMDA peptide is useful in the present invention if it includes amino acid substitutions, deletions, additions or rearrangements at sites such that antibodies raised against the analogue are still specific against the NMDA peptide.

Unless stated to the contrary, an NMDA analogue as used in this document refers to a sequence that has at least 80% amino acid identity with naturally occurring NMDA, although it could also contain at least 85%, 90%, or 95% identity. Amino acid identity is defined by an analogue comparison between the analogue and naturally occurring NMDA. The two amino acid sequences are aligned in such a way that maximizes the number of amino acids in common along the length of their sequences; gaps in either or both sequences are permitted in making the alignment in order to maximize the number of common amino acids. The percentage amino acid identity is the higher of the following two numbers: (1) the number of amino acids that the two peptides have in common with the alignment, divided by the number of amino acids in the NMDA analogue, multiplied by 100, or (2) the number of amino acids that the two peptides have in common with the alignment, divided by the number of amino acids in naturally occurring NMDA peptide, multiplied by 100.

NMDA derivatives include naturally occurring NMDA and NMDA analogues and fragments thereof that are chemically or enzymatically derivatized at one or more constituent amino acids, including side chain modifications, backbone modifications, and N- and C- terminal modifications, by for example acetylation, hydroxylation, methylation, amidation, phosphorylation or glycosylation. The term also includes NMDA salts such as zinc NMDA and ammonium NMDA.

A protein or peptide is measured "directly" in the sense that the protein or peptide is itself measured in the biological sample, as opposed to some other indirect measure of the protein or peptide such as autoantibodies to the protein or peptide; other peptide fragments from the same protein or subunit, or cDNA associated with the expression of the protein or peptide.

The term "antibody" is synonymous with "immunoglobulin." As used herein, the term "antibody" includes both the native antibody, monoclonally generated antibodies, polyclonally generated antibodies, recombinant DNA antibodies, and biologically active derivatives of antibodies, such as, for example, Fab', F(ab')₂ or Fv as well as single-domains and single-chain antibodies. A biologically active derivative of an antibody is included within this definition as long as it retains the ability to bind the specified antigen. Thus, an NR2 antibody has the ability to bind at least one NR2 peptide.

### Discussion

In one embodiment the invention provides a method for aiding in the assessment of the risk of stroke in an apparently healthy human subject prior to surgery comprising: (a) obtaining a test sample from the human subject; (b) analyzing the obtained test sample for the presence or amount of an NR2 antigen of an NR2 antibody, or a combination thereof; and comparing the result of step (b) with a corresponding reference amount of an NR2 antigen or NR2 antibody, or a combination thereof, wherein the corresponding reference amount is derived from a population of apparently healthy human subjects. Stated another way, the invention provides a method for predicting an adverse neurological event from surgery comprising: (a) providing a human patient scheduled for surgery; (b) measuring an initial level of circulating cerebral NMDA receptor peptides from a biological fluid in said patient; and (c) correlating said level to a risk for suffering an adverse neurological event from surgery.

When a patient is tested and has dangerous levels of NMDA receptor peptides or antibodies in his or her bloodstream, it is preferred to test the patient several more times before surgery and during surgery. In addition, because patients often do not suffer an adverse neurological event until shortly after the surgery, it is preferred to test these patients one or more additional times after surgery, within one or more of the following time periods: one hour, three hours, six hours, twelve hours, twenty four hours, three days, seven days, or thirty days. Thus, in one embodiment the invention further includes (a) measuring a subsequent level of circulating cerebral NMDA receptor peptides in said patient after said surgery; (b) determining whether there is a difference between said initial and subsequent levels; and (c) correlating a difference between said initial and subsequent levels to an adverse neurological event which is either occurring or likely to occur in the near future.

The methods can be performed on adults or children scheduled for surgery, and in a particularly important embodiment are performed in neonatal patients or infants who are at particular risk for neurological sequelae. The method is particularly useful when evaluating patients who are already predisposed to suffering a neurological event, such as patients with a history of diabetes, atherosclerosis, high blood pressure, or a previous suspected or confirmed TIA or stroke. The methods can also be used in conjunction with MMSA testing, before surgery, to predict the risk of an adverse neurological event. Preoperative decreased MMSA component scores for orientation, attention and recall have been associated with confusion and cerebrovascular events shortly after surgery.

The types of neurological events that can be predicted by the current invention are generally those induced by cerebral ischemia, and especially ischemic events that are caused by insufficient supplies of oxygen to the brain (as opposed to hemmorhagic events that occur when blood vessels are ruptured in the brain). These events can be focalized in a particular region of the brain, as occurs in stroke or TIA, or global, as occurs in delirium. The adverse neurological event may thus be characterized by confusion or may be diagnosed as a TIA or ischemic stroke. Oxygen supplies can be compromised due to the health condition of the patient (as in certain blood disorders such as anemia), but more commonly will be caused by the surgical event. The adverse neurological event is said to be "from" the surgery if the event occurs during surgery, or within thirty days after the surgery is completed, although the resulting adverse event could also be identified in a time frame of seven days, three days, two days or one day, if desired.

The prognostic methods of the present invention can predict the risk of adverse neurological events from any type of surgery, although traumatic surgeries that temporarily slow or halt the flow of oxygen to the brain will benefit most. For example, the method should be performed before any cardiovascular procedure that occludes or blocks normal blood circulation, that results in intraoperative micro-or macro-emboli, abnormal cerebral perfusion, reperfusion injury, or an inflammatory or neruhumoral response. The invention is especially useful in predicting the occurrence of adverse neurological events when a cardiopulmonary bypass is performed.

The testing methods can be performed based on measurements of any circulating NMDA receptor peptide, and they can be performed using any direct or indirect measurement technique. Thus, for example, the levels of circulating peptides can be measured by an indirect measure of antibodies to the peptides, CDNA expression encoding the peptides, or by measuring the peptides themselves. Therefore, in one embodiment the invention provides a method wherein said level is measured by contacting said biological fluid with an immobilized antibody or fragment thereof (i.e., one which is bound to a carrier such as a plate or bead or small particle), and directly measuring the level of one or more circulating cerebral NMDA receptor peptides. In another embodiment the invention provides a method wherein said level is measured by contacting said biological fluid with an immobilized cerebral NMDA. receptor peptide, and measuring the level of antibody, that binds to said immobilized peptide.

In addition, various subunits and fragments of the NMDA receptor may preferentially be measured. For example, the method is preferably performed by measuring NR2A peptides or NR2B peptides, and is even more preferably performed by measuring both. In one preferred embodiment, the method is performed by measuring one or more peptide sequences that are common to the native NR2A and NR2B sequences, one or more peptide sequences that are common to the N-terminal domain of native NR2A and NR2B sequences, or antibodies thereto. In another preferred embodiment, circulating peptides that comprise 2,7 and 14 kDa fragments of the N-terminal domain of the NR2A and NR2B subunits of a cerebral NMDA receptor are measured in the methods of the present invention.

The method can be performed using practically any biological fluid where circulating cerebral NMDA receptors, or markers of such receptors, are expressed or found, including blood, urine, blood plasma, blood serum, cerebrospinal fluid, saliva, perspiration or brain tissue. In a preferred embodiment the biological fluid is plasma or serum, and in an even more preferred embodiment the plasma or serum is diluted to a ratio of about 1:50.

In a preferred embodiment, a risk assessment is made based on a predetermined cutoff of peptide levels. Thus, for example, it has been experimentally and clinically shown that levels of NR2A/B N-terminal domain antibodies in plasma or serum that are greater than 2.0, 1.8, 1.5, or 1.0 ng/ml, or levels of NMDA peptides that correspond to NR2A/B N-terminal domain circulating peptides of greater than 200, 100, or 50 pg/ml, are remarkably predictive of an adverse neurological event in a patient undergoing surgery. In contrast, levels of NR2A/B N-terminal domain antibodies in plasma or serum that are less than 2.0, 1.8, 1.5, or 1.0 ng/ml, or levels of NMDA peptides that correspond to NR2A/B N-terminal domain circulating peptides of less than 200, 100, or 50 pg/ml, are remarkably predictive that an adverse neurological event will not occur during surgery. A preferred antibody cutoff is 2.0 ng/ml.

In a prospective clinical study reported in the examples hereto, patients with a preoperative positive peptide/antibody test (>200pg/ml and ≥2.0 ng/ml respectively) were nearly 18 times more likely to experience a post-operative neurological event than patients with a negative test (<2.0 ng/ml). According to clinical testing, women showed a higher risk ratio for neurological complications than men, and neurological adverse events were more common among elderly persons (>70 years). In addition, increased NIHSS scores combined with elevated (>2 ng/ml) concentrations of NR2 antibodies and peptide (>200 pg/ml) were shown to be predictive of TIA/stroke in patients undergoing surgery. However preoperative NIHSS alone are not predictive of adverse neurological events.

Based upon the results of the testing, various regimens can be implemented to decrease the risk of an adverse neurological event should surgery proceed as scheduled. For example, the method might further include, if the patient is at risk for suffering an adverse neurological event from surgery, (i) administering neuroprotective therapy to said patient, or (ii) implementing a monitoring program for monitoring the risk or occurrence of an adverse neurological event. Neuroprotective therapies include drug regimens, such as antiplatelet, anticoagulant, lipid-lowering and blood pressure lowering drugs. Alternatively, the method could further comprise, if the patient is at risk for suffering an adverse neurological event from surgery, optionally analyzing said patient for new infarction area defined by MRI, and performing neurosurgery or vascular surgery on said patient, such as carotid endarterectomy, direct endarterectomy, angioplasty and stent placement, extracranial-intracranial bypass, and vertebral artery transposition.

The method can be performed using any number of known diagnostic techniques, including immunoprecipitation (IP), indirect immuno-fluorescence (IIF), immunodot and immunoblotting (IB) (Western Blot), direct or indirect enzyme-linked immunosorbent assay (ELISA), radioimmunoassay (RIA), counter-immuno-electrophoresis (CIE), flow cytometry (FC), latex agglutination, lateral flow, fluorescence polarization assay, or microarray. In one particular embodiment, the invention is practiced using an immobilized solid phase for capturing and measuring the NMDA peptide marker. The invention thus provides a method for predicting an adverse neurological event from surgery comprising: (a) providing a human patient scheduled for surgery; (b) contacting a biological sample from said patient with an immobilized solid phase comprising a NR2 peptide or NR2 antibody, for a time sufficient to form a complex between said NR2 peptide or said NR2 antibody and NR2 antibody or NR2 peptide in said biological sample; (c) contacting said complex with an indicator reagent attached to a signal-generating compound to generate a signal; (d) measuring the signal generated; (e) correlating to signal generated to the level of said NR2 peptide or NR2 antibody in said sample; and (f) correlating the level of said NR2 peptide or NR2 antibody in said sample to said risk for an adverse neurological event. In a preferred embodiment, the indicator reagent comprises chicken anti-human or anti-human IgG attached to horseradish peroxidase.

In a preferred embodiment, the solid phase is a polymer matrix. More preferably, the polymer matrix is polyacrylate, polystyrene, or polypropylene. In one preferred embodiment the solid phase is a microplate. In another preferred embodiment, the solid phase is a nitrocellulose membrane or a charged nylon membrane.

In another embodiment, the method is performed using agglutination. In this embodiment, the invention provides a method for predicting an adverse neurological event from surgery comprising: (a) providing a human patient scheduled for surgery; (b) contacting a biological sample from said patient with an agglutinating carrier comprising a NR2 peptide or NR2 antibody, for a time sufficient to form an agglutination complex between said NR2 peptide or said NR2 antibody and NR2 antibody or NR2 peptide in said biological sample; (c) generating a signal from the agglutination; (d) correlating said signal to said levels of one or more markers of NR2 peptide; and (e) correlating the level of said NR2 peptide or NR2 antibody in said sample to said risk for an adverse neurological event In a preferred embodiment, the "sufficient time" is less than 30, 20, 15 or even 10 minutes.

Latex agglutination assays have been described in Beltz, G. A. et al., in Molecular Probes: Techniques and Medical Applications, A. Albertini et al., eds., Raven Press, New York, 1989. In the latex agglutination assay, antibody raised against a particular biomarker is immobilized on latex particles. A drop of the latex particles is added to an appropriate dilution of the serum to be tested and mixed by gentle rocking of the card. With samples lacking sufficient levels of the biomarkers, the latex particles remain in suspension and retain a smooth, milky appearance. However, if biomarkers reactive with the antibody are present, the latex particles clump into visibly detectable aggregates.

An agglutination assay can also be used to detect biomarkers wherein the corresponding antibody is immobilized on a suitable particle other than latex beads, for example, on gelatin, red blood cells, nylon, liposomes, gold particles, etc. The presence of antibodies in the assay causes agglutination, similar to that of a precipitation reaction, which can then be detected by such techniques as nephelometry, turbidity, infrared spectrometry, visual inspection, colorimetry, and the like.

The term latex agglutination is employed generically herein to refer to any method based upon the formation of detectable agglutination, and is not limited to the use of latex as the immunosorbent substrate. While preferred substrates for the agglutination are latex based, such as polystyrene and polypropylene, particularly polystyrene, other well-known substrates include beads formed from glass, paper, dextran, and nylon. The immobilized antibodies may be covalently, ionically, or physically bound to the solid-phase immunoadsorbent, by techniques such as covalent bonding via an amide or ester linkage, ionic attraction, or by adsorption. Those skilled in the art will know many other suitable carriers for binding antibodies, or will be able to ascertain such, using routine experimentation.

Conventional methods can be used to prepare antibodies for use in the present invention. For example, by using a peptide of a NMDA protein, polyclonal antisera or monoclonal antibodies can be made using standard methods. A mammal, (e.g., a mouse, hamster, or rabbit) can be immunized with an immunogenic form of the peptide (preferably the NR2A and/or NR2B receptor, an antigenic determinant of the NR2A and/or NR2B receptor, or an analogue or derivative thereof) which elicits an antibody response in the mammal. Techniques for conferring immunogenicity on a peptide include conjugation to carriers or other techniques well known in the art. For example, the peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be administered and, if desired, polyclonal antibodies isolated from the sera.

To produce monoclonal antibodies, antibody producing cells (lymphocytes) can be harvested from an immunized animal and fused with myeloma cells by standard somatic cell fusion procedures thus immortalizing these cells and yielding hybridoma cells. Such techniques are well known in the art, (e.g., the hybridoma technique originally developed by Kohler and Milstein (Nature 256, 495-497 (1975)) as well as other techniques such as the human B-cell hybridoma technique (Kozbor et al., Immunol. Today 4, 72 (1983)), the EBV-hybridoma technique to produce human monoclonal antibodies (Cole et al. Monoclonal Antibodies in Cancer Therapy (1985) Allen R. Bliss, Inc., pages 77-96), and screening of combinatorial antibody libraries (Huse et al., Science 246, 1275 (1989)]. Hybridoma cells can be screened immunochemically for production of antibodies specifically reactive with the peptide and the monoclonal antibodies can be isolated. Therefore, the invention also contemplates hybridoma cells secreting monoclonal antibodies with specificity for NR2A or NR2B NMDA proteins or fragments thereof as described herein.

In one embodiment the method is practiced using a kit that has been calibrated at the factory based upon antibodies or peptides purified from human blood. Therefore, in another embodiment the invention is practiced under the following conditions: (a) NR2 antibody or peptide levels in said biological fluid are measured using a diagnostic kit; (b) said diagnostic kit comprises bound NR2 peptides or antibodies; and (c) said kit is manufactured against an antibody or peptide standard comprising a fraction of immunoglobulins G or peptides purified from human blood.

In addition, the method can be practiced using commercially available chemiluminescence techniques. For example, the method could employ a two-site sandwich immunoassay using direct chemiluminescent technology, using constant amounts of two monoclonal antibodies. The first antibody, in a fluid reagent, could be an acridinium ester labeled monoclonal mouse anti-human NMDA receptor peptide BNP (F(ab')₂ fragment specific to a first portion of the peptide. The second antibody, in the solid phase, could be a biotinylated monoclonal mouse anti-human antibody specific to another portion of the peptide, which could be coupled to streptavidin-functionalized magnetic particles. An immuno-complex would be formed by mixing a patient sample and the two antibodies. After any unbound antibody conjugates are washed away, the chemiluminescence of the immuno-complex signal could then be measured using a luminometer.

The immunosorbent of the present invention for measuring levels of autoantibody can be produced as follows. A fragment of the receptor protein is fixed, preferably by covalent bond or an ionic bond, on a suitable carrier such as polystyrene or nitrocellulose. If the standard polystyrene plate for immunological examinations is employed, it is first subjected to the nitration procedure, whereby free nitrogroups are formed on the plate surface, which are reduced to amino groups and activated with glutaric dialdehyde serving as a linker. Next the thus-activated plate is incubated with about 2 to 50 nM of the target peptide for the purpose of chemically fixing the respective immunogenic fragment of the receptor protein for a time and at a temperature sufficient to assure fixation (i.e. for about 16 hours at 4°C).

It is also practicable to produce the immunosorbent by fixing the respective fragment of the receptor protein on nitrocellulose strips by virtue of ionic interaction. The respective fragment of the receptor protein isolated from the mammals' brain is applied to nitrocellulose and incubated for 15 min at 37°C. Then nitrocellulose is washed with a 0.5 % solution of Tween-20, and the resultant immunosobent is dried at room temperature and stored in a dry place for one year period.

### EXAMPLES

The following examples are put forth so as to provide those of ordinary skill in the art with a complete disclosure and description of how the compounds claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at room temperature, and pressure is at or near atmospheric.

### EXAMPLE 1. NMDA PEPTIDE TEST

A preferred NR2 peptide test is a latex agglutination immunoassay for the qualitative determination of NR2 peptide in the blood. Blood samples are mixed with antibody coupled to latex beads and agglutination is visually detected within 10 minutes. After addition of a blood sample to the sample port of the test device, the red blood cells are separated from plasma by a filter. A predetermined quantity of plasma moves by capillary action into a reaction chamber, where it reacts with latex reagent (antibody-coated latex beads) to form complexes that can be detected visually. In the examples reported herein, the test uses calibrators set from 100-5000 pg/ml and standards with "low" (<200pg/ml) and "high" (1000 pg/ml) values of NR2 peptide.

### EXAMPLE 2. NMDA ANTIBODY TEST

A rapid assay of NR2 antibody assay (CIS-LA antibody test) is based on a latex agglutination technique. The CIS-LA antibody assay employs triple concave slides with a built-in magnification device to detect the reaction visually, providing an immediate "yes" or "no" response. In this assay, serum samples are mixed with NR2 peptide coupled with colored latex particles and agglutination is visually detected through built-in magnification device within 2 to 5 minutes or is indicated using nephelometer. The IgG concentrations in patient samples is expressed in ng/ml according to a calibration curve from a set of calibrators of 0-100 ng/ml and standards with "low" (<2.0 ng/ml) and "high" (6 ng/ml) values of NR2 antibodies.

### EXAMPLE 3. EVALUATION OF NR2 ANTIBODIES IN ADULT SURGERY PATIENTS

Thirty adult patients scheduled for CPB surgery were evaluated for NR2 antibody levels in serum before the surgery, 24 hours after surgery, and 48 hour after surgery, and adverse events recorded. Results are presented below in Table 1.

**Table 1**

| No. | NR2 antibody, mean ± SD, ng/ml | | | Surgery type | Adverse events |
|---|---|---|---|---|---|
| | pre | 24h post | 48 h post | | |
| 1 | 1.70 ± 0.14 | 1.58 ± 0.12 | 1.55 ± 0.13 | Valve | 1^{st} postop hemorr. |
| 2 | 1.79 ± 0.10 | 1.64 ± 0.03 | 2.76 ± 0.08 | CABG | 1^{st}-3^{rd} postop, C |
| 3 | 2.25 ± 0.02 | 1.81 | 2.14 ± 0.03 | CABG+valve | 2^{nd} postop, C (hr) |
| 4 | 2.00 ± 0.04 | 3.23 ±0.09 | 3.34 ± 0.15 | CABG | 3^{rd}-5^{th} postop, C |
| 5 | 3.9 ± 0.09 | 2.32 ± 0.06 | 2.70 ± 0.15 | CABG | 2^{nd} postop, IS |
| 6 | 5.4 ± 0.12 | 4.65 ± 0.09 | 4.60 ± 0.25 | CABG+valve | 2^{nd} postop, C&D, *DM* |
| 7 | 2.29 ±0.09 | 2.05 ±0.02 | 2.20 ±0.02 | CABG | Preop, *C |
| 8 | 1.69 ±0.02 | 1.35 ± 0.004 | 122 ± 0.07 | CABG+valve | 3^{rd} postop, C (hr) |
| 9 | 1.94 ± 0.03 | 1.69 ± 0.005 | 1.38 ± 0.03 | Valve | 2^{nd} postop, C (4hr) |
| 10 | 1.78 ± 0.14 | 1.89 ± 0.04 | 1.65 | CABG+valve | 1^{st} postop, **, *DM* |
| 11 | 1.70 ± 0.03 | 2.00 ± 0.006 | 2.35 ± 0.24 | CABG+valve | 2^{nd} postop, C&D, *DM* |
| 12 | 1.40 ± 0.03 | 1.38 ±0.03 | 1.39 ± 0.06 | CABG+valve | - |
| 13 | 2.11 ± 0.03 | 1.81 ± 0.02 | 1.93 ± 0.16 | CABG | - |
| 14 | 1.79 ± 0.03 | 1.40 ± 0.07 | 1.39 ± 0.02 | CABG | 1^{st} postop, C (24hr) |
| 15 | 1.47 ± 0.02 | 1.30 ± 0.08 | 1.31 ± 0.07 | CABG+valve | 25^{th} postop, depress. |
| 16 | 0.70 ± 0.09 | 0.69 ± 0.06 | 0.60 ± 0.03 | CABG | 2-3^{rd} postop, seizures |
| 17 | 1.16 ± 0.09 | 1.09 ± 0.06 | 1.14 ± 0.06 | CABG | 2^{nd} postop, D |
| 18 | 1.16 ± 0.004 | 0.78 ± 0.03 | 0.60 ± 0.01 | CABG | 1^{st} postop, anxiety,C |
| 19 | 1.35 ± 0.05 | 1.10 ± 0.015 | 1.16 ± 0.04 | CABG | 2^{nd} postop, C |
| 20 | 1.21 ± 0.005 | 0.92 ± 0.09 | 0.82 ± 0.05 | CABG | 2^{nd} postop, C |
| 21 | 2.5 ± 0.08 | 1.86 ± 0.04 | 1.73 ± 0.03 | CABG | Preop, agitation |
| 22 | 0.99 ± 0.01 | 0.78 ± 0.07 | 0.80 ± 0.06 | CABG | 1^{st} post, agit.&insom |
| 23 | 1.00 | 0.92 ± 0.02 | 0.82 | CABG | 3^{rd} postop, C |
| 24 | 1.10 ± 0.18 | 1.15 ± 0.05 | 0.98 ± 0.004 | CABG | 1^{st} postop, anxiety |
| 25 | 1.04 ± 0.02 | 0.64 ± 0.03 | 0.61 ± 0.02 | CABG | 1^{st} postop, anxiety |
| 26 | 0.98 ± 0.03 | 0.81 ± 0.05 | 0.70 ± 0.004 | CABG | 7^{th} postop, anxiety |
| 27 | 0.91 ± 0.005 | 0.82 ± 0.08 | 0.79 ± 0.01 | CABG | 2^{nd} postop, C |
| 28 | 0.92 ± 0.03 | 1.15 ± 0.09 | 0.69 ± 0.01 | CABG | 1^{st} postop, C |
| 29 | 1.04 ± 0.03 | 0.90 ± 0.01 | 0.79 ± 0.03 | CABG+valve | Preop, agitation |
| 30 | 1.10 ± 0.11 | 0.82 ± 0.09 | No sample | CABG+valve | 1^{st} postop, agitation |
| 31 | 0.72 ± 0.004 | 0.73 ± 0.004 | 0.68 ± 0.01 | CABG+valve | 1-4^{th} postop, seizures |
| 32 | 0.73 ± 0.04 | 0.68 ± 0.02 | 0.70 ± 0.04 | valve | Preop, agitation, 5-11^{th} encephalo |

| | | | | | |
|---|---|---|---|---|---|
| IS - ischemic stroke; C - confusion; D - disorientation; *DM* - diabetes mellitus * impaired swallow; ** change in mental status | | | | | |

### EXAMPLE 4. EVALUATION OF NR2 PEPTIDE AND ANTIBODY LEVELS DURING AND AFTER SURGERY

More than 32,000 infants (one out of every 125 to 150) are born with congenital heart defects each year in the United States. JT patient (date of birth-06/25/04) underwent surgery to repair congenital heart defect using CPB on December 7, 2004. Neurodevelopment scores (Mullen Scales of Early Learning, MSEL) assessing mental and motor ability were evaluated pre-operatively, and are presented below in Table 2. This infant showed low scores and depressed visual, language abilities and motor weakness.

**Table 2**

| Scale | Scores | Percentile |
|---|---|---|
| Early Learning Composite | Standard Score of 80 | 9 |
| Gross Motor Scale | t-score 28 | 1 |
| Visual Reception | t-score 45 | 31 |
| Fine Motor | t-score 34 | 5 |
| Receptive Language | t-score 37 | 10 |
| Expressive Language | t-score 42 | 21 |

The results of NR2 peptide/antibodies monitoring in blood of JT patient are presented in Table 3. Abnormally high levels of the peptide and antibodies were detected from the intubation and up to the end of CPB. The elevated above the norm pre-op NR2 peptide/antibodies data demonstrated a good correlation with pre-op MSEL scores and confirmed the brain injury and neurological adverse event due to CPB. An age-matched control group (n=7) of infants without congenital heart defects had NR2 peptide of 0.2-0.4 ng/ml and NR2 antibodies of 0.4-0.8 ng/ml.

**Table 3**

| Date | Time | Surgery | NR2 peptide, ng/ml | NR2 antibody ng/ml |
|---|---|---|---|---|
| 12/7/2004 | 7:55 | incubation | 9.5 ± 0.07 | 1.4 ± 0.12 |
| 12/7/2004 | 10:14 | CPB | 10.0 ± 0.01 | 3.1 ± 1.02 |
| 12/7/2004 | 11:53 | 1h on CPB | 7.0 ± 0.33 | 1.2 ± 0.27 |
| 12/7/2004 | 14:35 | end of CPB | 2.7 ± 0.09 | 1.0 ± 0.02 |
| 12/8/2004 | 11:30 | 24 h post | 2.5 ± 0.15 | 1.2 ± 0.01 |
| 12/9/2004 | 9:30 | 48 h post | 4.6 ± 0.05 | 1.7 ± 0.07 |

### EXAMPLE 5. PERFORMANCE CHARACTERISTICS OF CIS-LA ANTIBODY TEST

Table 4 presents the results of a study undertaken in CPB patients to evaluate the capacity of pre-op NR2 Ab levels for predicting the likelihood of adverse neurological events. The results are presented for different cut offs of NR2 Ab levels in patient se4rum, and subdivided based on the presence or absence of a post-operative adverse event. Patients were deemed to have suffered an adverse neurological event if, within twenty eight days of the CBP surgery, the patient suffered from confusion, TIA or stroke were detected, based on an NIHSS score of greater than nine. Patients who did not suffer any neurological event were assigned to the "No Neuro Event" group.

**Table 4**

| Pre-Op NR2 Ab | Neuro Event n/N (%) | No Neuro Event n/N (%) |
|---|---|---|
| < 1.5 ng/mL | 7/213 (3.3%) | 206/213 (96.7%) |
| 1.5 to < 2.0 ng/mL | 12/159 (7.6%) | 147/159 (92.5%) |
| ≥ 2.0 ng/mL | 25/26 (96.2%) | 1/26 (3.9%) |

Table 5 provides a detailed analysis of six different cut offs for NR2 Ab concentrations from 1.5 to 2.0 ng/ml, and demonstrates the efficacy of each cutoff at predicting adverse neurological events. Although the event rate increases in the cut offs from 1.5 to 2.0 for both groups, it increases faster in the "Neuro Event" group. Therefore, the risk ratio increases significantly over the analyzed range with the best risk ratio corresponding to 2.0 ng/mL (ClinChem, 2003). 96.0% (24/25) of patients with NR2 Ab concentrations ≥2.0 ng/ml preoperatively had neurological complications within 48 hours post-CPB, vs. only 5.4 % of patients with NR2 Ab concentrations <2.0 ng/ml, resulting in a 17.9-fold increase (95% CI, 11.6-27.6) in the ability of the marker to predict postoperative neurological adverse events.

**Table 5**

| Pre-Op NR2 Ab | Neuro Event n/N (%) | No Neuro Event n/N (%) | Risk Ratio¹ (Lower 95% Bound)² |
|---|---|---|---|
| < 1.5.ng/mL | 8/214 (3.7%) | 206/214 (96.3%) | 5.2 |
| ≥ 1.5 ng/mL | 36/184 (19.6%) | 148/184 (80.0%) | (2.8) |
| | | | |
| < 1.6 ng/mL | 10/288 (3.5%) | 278/288 (96.5%) | 8.9 |
| ≥ 1.6 ng/mL | 34/110 (30.9%) | 76/110 (69.1%) | (5.1) |
| | | | |
| < 1.7 ng/mL | 12/319 (3.8%) | 307/319 (96.2%) | 10.8 |
| ≥ 1.7 ng/mL | 32/79 (40.5%) | 47/79 (59.5%) | (6.4) |
| | | | |
| < 1.8 ng/mL | 17/351 (4.8%) | 334/351 (95.2%) | 11.9 |
| ≥ 1.8 ng/mL | 27/47 (57.5%) | 20/47 (42.6%) | (7.6) |
| | | | |
| < 1.9 ng/mL | 19/364 (5.2%) | 345/364 (94.8%) | 14.1 |
| ≥ 1.9 ng/mL | 25/34 (73.5%) | 9/34 (26.5%) | (9.4) |
| | | | |
| < 2.0 ng/mL | 20/373 (5.4%) | 353/373 (94.6%) | 17.9 |
| ≥ 2.0 ng/mL | 24/25 (96.0%) | 1/25 (4.0%) | (12.4) |

| | | | |
|---|---|---|---|
| ¹Ratio of Event rate among patients with a positive pre-op NR2 Ab divided by event rate among patients with a negative pre-op NR2 Ab; ²Lower one-sided 95% confidence bound on the risk ratio | | | |

Presently at least 30 % of patients who undergo cardiac surgery have neurocognitive deficit post-operatively. Based on the obtained likelihood ratio ofneuro event of 17.9, NR2 antibodies concentrations greater that 2.0 ng/ml detected pre-operatively will predict neurological complications in 89 % of patients after surgery.

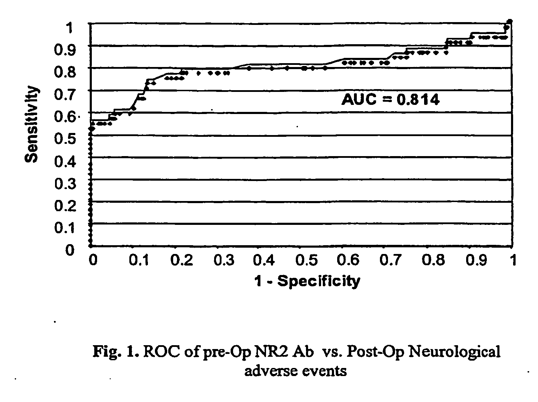

Figure 1 presents three ROC curves based on data for the NR2 test. The area under the curve for pre-op NR2 Ab indicates that the NR2 Ab marker has a high predictive ability (AUC=0.814) for neurological adverse events before surgery.

### EXAMPLE 6. ANALYSES OF PRE-OP AND POST-OP DISTRIBUTION OF NR2 ANTIBODIES

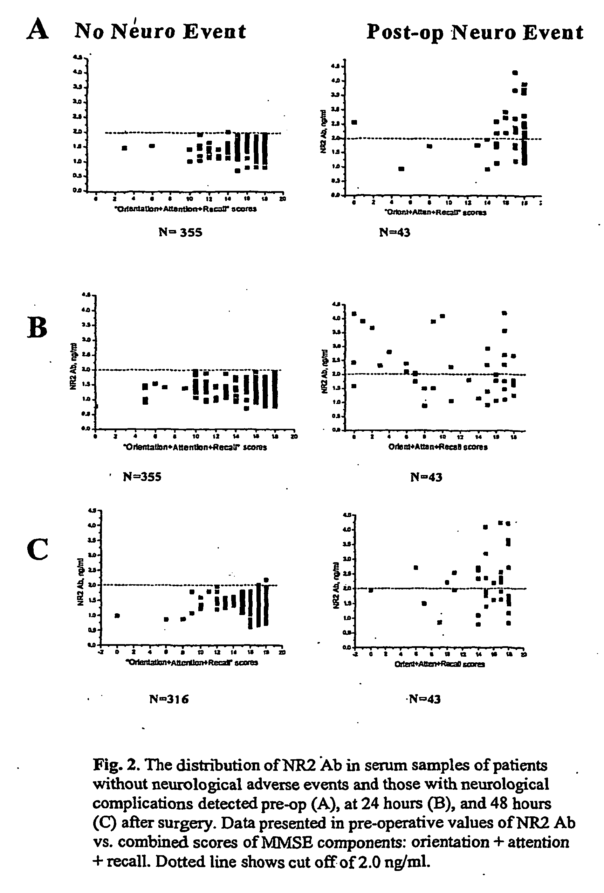

The high predictive value of the NR2 Ab marker for TIA/stroke before CPB is illustrated in Figure 2. The concentrations of NR2 Ab in serum samples from patients with no adverse events (neurocode 0) remained under the cut off of 2.0 ng/ml at all time points during the study. In contrast, most patients with neurological adverse events (NIHSS scores > 9) had increased NR2 Ab values above the cut off of 2.0 ng/ml pre-operatively, and at 24 hours and 48 hours after the procedure.

Detailed analyses of NR2 Ab distributions among the patient group pre-operatively, 24 and 48 hours post-operatively showed that NR2 Ab can reliably reveal patients with neurological complications before surgery in patients with neurological complications while patients without neurological complications had NR2 Ab values under the cut off. The NR2 Ab biomarker was sensitive to a decrease (i.e. worsening) in NIHSS scores at 24 h after surgery.

### CONCLUSION

Throughout this application, various publications are referenced.

## Claims

1. A method for aiding in the assessment of the risk of an adverse neurological event in an apparently healthy human subject prior to surgery comprising:
a) analyzing a blood serum sample obtained from a human subject for the initial level of an NMDA receptor peptide or antibody, or a combination thereof;
b) comparing the result of step (a) with a corresponding reference amount of an NMDA receptor peptide or antibody, or a combination thereof,
wherein an initial level of antibodies corresponding to NR2A and/or B N-terminal domain antibodies in said blood serum of greater than 2.0 ng/ml, or levels of NMDA peptides corresponding to NR2A and/or B N-terminal domain circulating peptides of greater than 200 pg/ml, correlates to a risk for suffering said neurological event; and an initial level of antibodies corresponding to NR2A and/or B N-terminal domain antibodies in said fluid of less than 2.0 ng/ml or levels of NMDA peptides corresponding to NR2A and/or B N-terminal domain circulating peptides of less than 200 pg/ml, does not correlate to a risk for suffering said neurological event.

2. The method of claim 1, wherein said adverse neurological event occurs during surgery, or within 72 hours of surgery.

3. The method of claim 1, wherein said surgery comprises anesthesia.

4. The method of claim 1, wherein said surgery comprises a cardiopulmonary bypass.

5. The method of claim 1, wherein said patient is a neonate or infant at risk for neurological sequelae.

6. The method of claim 1, wherein said NMDA receptor peptides comprise the N-terminal domain of an NR2A subunit of a cerebral NMDA receptor, the N-terminal domain of an NR2B subunit of a cerebral NMDA receptor, or a combination thereof.

7. The method of claim 1, wherein said human patient is diagnosed as having diabetes, atherosclerosis, or a previous suspected stroke or TIA.

8. The method of claim 1 performed by direct or indirect ELISA, RIA, immunodot, immunoblot, latex agglutination, lateral flow, fluorescent polarization, or microarray.

## Patentansprüche

1. Verfahren zur Unterstützung bei der Beurteilung des Risikos eines nachteiligen neurologischen Ereignisses in einem scheinbar gesunden menschlichen Wesen vor der Operation, welches umfasst, dass
a) der anfängliche NMDA-Rezeptor-Peptid- oder -Antikörperspiegel oder ein Spiegel einer Kombination davon in einer Blutserumprobe, die von einem menschlichen Wesen erhalten wird, analysiert wird;
b) das Ergebnis aus Schritt (a) mit einer entsprechenden Referenzmenge eines NMDA-Rezeptor-Peptids oder eines Antikörpers, oder einer Kombination davon, verglichen wird,
wobei ein anfänglicher Spiegel von Antikörpern, die NR2A und/oder B N-terminale Domäne-Antikörpern entsprechen, in dem Blutserum von größer als 2,0 ng/ml, oder Spiegel von NMDA-Peptiden, die NR2A und/oder B N-terminale Domäne zirkulierenden Peptiden entsprechen, von größer als 200 pg/ml, mit einem Risiko für das Erleiden des neurologischen Ereignis korrelieren; und wobei ein anfänglicher Spiegel von Antikörpern, die NR2A und/oder B N-terminale Domäne-Antikörpern entsprechen, in der Flüssigkeit von weniger als 2,0 ng/ml oder Spiegel von NMDA-Peptiden, die NR2A und/oder B N- terminale Domäne zirkulierenden Peptiden entsprechen, von weniger als 200 pg/ml, nicht mit einem Risiko für das Erleiden des neurologischen Ereignisses korrelieren.

2. Verfahren nach Anspruch 1, wobei das nachteilige neurologische Ereignis während der Operation, oder innerhalb von 72 Stunden nach der Operation auftritt.

3. Verfahren nach Anspruch 1, wobei die Operation Anästhesie umfasst.

4. Verfahren nach Anspruch 1, wobei die Operation einen Herz-Lungen-Bypass umfasst.

5. Verfahren nach Anspruch 1, wobei der Patient ein Neugeborenes oder Kleinkind mit einem Risiko für neurologische Spätfolgen ist.

6. Verfahren nach Anspruch 1, wobei die NMDA-Rezeptor-Peptide die N-terminale Domäne einer NR2A-Untereinheit eines zerebralen NMDA-Rezeptors, die N-terminale Domäne einer NR2B-Untereinheit eines zerebralen NMDA-Rezeptors oder eine Kombination davon aufweisen.

7. Verfahren nach Anspruch 1, wobei diagnostiziert wird, dass der menschliche Patient Diabetes, Atherosklerose, oder einen vorherigen mutmaßlichen Schlaganfall oder Transitorische ischämische Attacke (TIA) hat.

8. Verfahren nach Anspruch 1, welches durch direkten oder indirekten ELISA, RIA, lmmunodot, lmmunoblot, Latex-Agglutination, Lateral-Flow, Fluoreszenz-Polarisation oder Microarray durchgeführt wird.

## Revendications

1. Procédé d'aide à l'évaluation du risque d'un événement neurologique indésirable chez un sujet humain apparemment en bonne santé avant une opération comprenant :
a) une analyse d'un échantillon de sérum sanguin obtenu auprès d'un sujet humain en ce qui concerne le niveau initial d'un peptide ou d'un anticorps de récepteur de N-Méthyl-D-Aspartate (NMDA), ou une combinaison de ceux-ci ;
b) une comparaison du résultat de l'étape (a) à une quantité de référence correspondante d'un peptide ou d'un anticorps de récepteur de NMDA, ou d'une combinaison de ceux-ci,
où un niveau initial d'anticorps correspondant à des anticorps de domaine N-terminal NR2A et/ou NR2B dans ledit sérum sanguin supérieur à 2,0 ng/ml, ou des niveaux de peptides de NMDA correspondant à des peptides de circulation de domaine N-terminal NR2A et/ou NR2B supérieurs à 200 pg/ml, est ou sont corrélés à un risque de souffrir dudit événement neurologique ; et un niveau initial d'anticorps correspondant à des anticorps de domaine N-terminal NR2A et/ou NR2B dans ledit fluide inférieur à 2,0 ng/ml, ou des niveaux de peptides de NMDA correspondant à des peptides de circulation de domaine N-terminal NR2A et/ou NR2B inférieurs à 200 pg/ml, n'est pas corrélé ou ne sont pas corrélés à un risque de souffrir dudit événement neurologique.

2. Procédé selon la revendication 1, dans lequel ledit événement neurologique indésirable survient au cours d'une opération, ou dans les 72 heures suivant l'opération.

3. Procédé selon la revendication 1, dans lequel ladite opération comprend une anesthésie.

4. Procédé selon la revendication 1, dans lequel ladite opération comprend une dérivation cardiopulmonaire.

5. Procédé selon la revendication 1, dans lequel ledit patient est un nouveau-né ou un enfant présentant un risque de séquelles neurologiques.

6. Procédé selon la revendication 1, dans lequel lesdits peptides de récepteur de NMDA comprennent le domaine N-terminal d'une sous-unité NR2A d'un récepteur de NMDA cérébral, le domaine N-terminal d'une sous-unité NR2B d'un récepteur de NMDA cérébral, ou une combinaison de ceux-ci.

7. Procédé selon la revendication 1, dans lequel ledit patient humain est diagnostiqué comme présentant des diabètes, une athérosclérose, ou une attaque préalable soupçonnée ou un accès ischémique transitoire cérébral.

8. Procédé selon la revendication 1, effectué par dosage ELISA direct ou indirect, par immuno-empreinte, par immunotransfert, par test d'agglutination au latex, par immunochromatographie sur membrane, par polarisation fluorescente, ou par microréseau d'ADN.
